# EUROPEAN PATENT APPLICATION

(11) **EP 4 755 417 A1**
(43) Date of publication of application: **10.06.2026**
(21) Application number: 24849490.8
(22) Date of filing: 25.07.2024
(51) Int. Cl.: A61M 5/30, A61M 5/315, A61M 35/00, A61M 37/00, A61M 5/31

(54) **MODULAR PRESSURE TRANSFER UNIT AND DRUG DELIVERY DEVICE COMPRISING SAME**

(30) Priority: 28.07.2023 KR 20230098746; 17.04.2024 KR 20240051096; 05.06.2024 KR 20240073431; 24.07.2024 KR 20240097882
(71) Applicant: Jskbiomed Inc., Daejeon (KR)
(72) Inventor: JEON, Jinwoo, Sejong 30101 (KR); BYUN, Jungin, Sejong 30052 (KR); BANG, Hyunseok, Daejeon 34310 (KR)
(74) Representative: Jung, Minkyu
(86) International application number: PCT/KR2024/010859
(87) International publication number: WO 2025/028921

(57) **Abstract**

Provided are a modular pressure transfer unit that includes a housing configured to form a sealed internal space, wherein an inside of the housing is filled with a fluid, a first elastic layer formed of a material having elasticity, coupled to the housing, and configured to transfer pressure applied from one side of an outside of the housing to the fluid, and a second elastic layer formed of a material having elasticity, coupled to the housing, and configured to transfer the pressure transferred through the fluid to another side of the outside of the housing.

## Description

### TECHNICAL FIELD

The present invention relates to a modular pressure transfer unit and a drug delivery device including the same, and more particularly, to a modular pressure transfer unit having a plurality of elastic layers and capable of transferring pressure from one side to another side through a fluid filled therein, and a drug delivery device including the same.

### BACKGROUND ART

Drug delivery devices are intended to deliver drugs to a human body for various purposes, such as treating diseases or wounds of the human body or for cosmetic purposes, and an injection method using a needle is most commonly used due to its stability and efficiency.

However, regarding the drug delivery device using a needle, while it is clear that an intended dose is administered into the human body, since most targets are fine and thin structures, it is unknown whether the drug accurately and effectively reaches or reacts with the target. In addition, it may cause a patient to have needle phobia due to pain during injection, and also has problems such as a risk of infection due to reuse of the needle and generation of a large amount of medical waste.

Accordingly, development of structures for delivering a drug to a human body without a needle is continuing.

### DESCRIPTION OF EMBODIMENTS

### TECHNICAL PROBLEM

The technical problem to be achieved by the present invention is to provide a modular pressure transfer unit having a plurality of elastic layers and capable of transferring pressure from one side to another side through a fluid filled therein, and a drug delivery device including the same.

### SOLUTION TO PROBLEM

According to an embodiment of the present invention, a modular pressure transfer unit may comprise a housing configured to form a sealed internal space, wherein an inside of the housing is filled with a fluid, a first elastic layer formed of a material having elasticity, coupled to the housing, and configured to transfer pressure applied from one side of an outside of the housing to the fluid, and a second elastic layer formed of a material having elasticity, coupled to the housing, and configured to transfer the pressure transferred through the fluid to another side of the outside of the housing.

According to an embodiment, the housing may comprise a first sub-housing in which the first elastic layer is formed, and a second sub-housing in which the second elastic layer is formed.

According to an embodiment, the second sub-housing may comprise a support portion in which the second elastic layer is formed, an insertion portion connected to the support portion and configured such that at least a part thereof is inserted into an inside of the first sub-housing, and a fixing portion connected to the insertion portion and configured to be caught and fixed by a fixing pin in a state where the insertion portion is fully inserted into the inside of the first sub-housing.

According to an embodiment, a packing portion may be configured to seal a gap between the insertion portion and an inner surface of the first sub-housing is formed on an outer surface of the insertion portion.

According to an embodiment, the fixing portion may comprise an extension portion connected to the insertion portion, and a protrusion connected to the extension portion and configured to form a engagement protrusion on which the fixing pin is caught.

According to an embodiment, the first elastic layer may be configured to expand toward an interior of the housing by the pressure applied from the outside of the housing, and wherein the second elastic layer may be configured to expand toward an exterior of the housing by the pressure transferred through the fluid.

According to an embodiment, the modular pressure transfer unit may further comprise an identification tag configured to store identification information of the modular pressure transfer unit.

According to an embodiment, the first elastic layer may be formed in a plurality of regions on an upper surface of the housing.

According to an embodiment, the plurality of regions may be located at positions symmetric to each other with respect to a center point of the upper surface of the housing.

According to an embodiment of the present invention, A drug delivery device may comprise a driving unit, a modular pressure transfer unit coupled to the driving unit and configured to transfer pressure applied from the driving unit, and a nozzle unit coupled to the modular pressure transfer unit and configured to inject a drug solution using the pressure transferred by the modular pressure transfer unit, wherein the modular pressure transfer unit may comprise a housing configured to form a sealed internal space, wherein an inside of the housing is filled with a fluid, a first elastic layer formed of a material having elasticity, coupled to the housing, and configured to transfer the pressure applied from the driving unit to the fluid, and a second elastic layer formed of a material having elasticity, coupled to the housing, and configured to transfer the pressure transferred through the fluid to the drug solution inside the nozzle unit.

According to an embodiment, the driving unit may comprise a pressing portion configured to apply pressure to the first elastic layer, and a driving device configured to control an operation of the pressing portion.

According to an embodiment, the drug delivery device may further comprise a coupling detection device configured to detect a coupling state between the driving unit and the modular pressure transfer unit.

According to an embodiment, the coupling detection device may be configured to collect identification information of the coupled modular pressure transfer unit.

According to an embodiment, the driving device may be configured to, when the modular pressure transfer unit is detected as being in a coupled state by the coupling detection device, receive the collected identification information and determine a total usage amount of the modular pressure transfer unit corresponding to the identification information.

According to an embodiment, the total usage amount of the modular pressure transfer unit may correspond to a cumulative value of pressure determined based on a number of times of pressing the first elastic layer by the pressing portion and a pressure at a time of pressing.

### ADVANTAGEOUS EFFECTS OF DISCLOSURE

A device according to an embodiment of the present invention may control a ejection amount and a ejection speed of a drug solution regularly and stably by providing a plurality of elastic layers and transferring pressure applied from an outside to the drug solution through a fluid filled therein.

In addition, the device according to an embodiment of the present invention may configure a structure for pressure transfer in a modular type, thereby allowing a pressure transfer unit to be partially replaced in a drug delivery device, so that elastic layers for which durability management is important can be stably managed.

In addition, the device according to an embodiment of the present invention may maintain a drug solution side elastic layer in a non-expanded state before use, and maintain the drug solution side elastic layer in an expanded state toward the drug solution through a process of fastening a part of the device to a fixing pin during use, thereby securing both ease of storage and efficiency of pressure transfer.

In addition, the device according to an embodiment of the present invention may efficiently manage a replacement cycle of a modular pressure transfer unit by managing a usage amount after a time point when the modular pressure transfer unit is coupled for each modular pressure transfer unit.

### BRIEF DESCRIPTION OF DRAWINGS

A brief description of each drawing is provided to more fully understand drawings recited in the detailed description of the present invention.
FIG. 1 is a conceptual diagram of a drug delivery device according to an embodiment of the present invention.
FIGS. 2 and 3 are diagrams for explaining an operation process of the drug delivery device shown in FIG. 1.
FIG. 4 is a diagram illustrating detailed components of the drug delivery device shown in FIG. 1.
FIG. 5 is a block diagram according to an embodiment of the driving device shown in FIG. 4.
FIGS. 6 and 7 are diagrams showing a detailed structure of the modular pressure transfer unit shown in FIG. 4.
FIGS. 8 and 9 are diagrams showing states before use and a state for use of the modular pressure transfer unit shown in FIG. 6.
FIGS. 10 and 11 are diagrams showing modified embodiments of the modular pressure transfer unit shown in FIG. 4.

### MODE OF DISCLOSURE

The technical idea of the present invention may be modified in various ways and may have various embodiments, so specific embodiments will be illustrated in the drawings and described in detail. However, this is not intended to limit the technical idea of the present invention to particular modes of practice, and it is to be appreciated that all changes, equivalents, and substitutes that do not depart from the technical idea and scope of the present invention are encompassed in the present invention.

In the description of the technical idea of the present invention, certain detailed explanations of related art are omitted when it is deemed that they may unnecessarily obscure the essence of the present invention. In addition, numeral figures (e.g., first, second, and the like) used during describing the specification are just identification symbols for distinguishing one element from another element.

Further, in the specification, if it is described that one component is "connected" or "accesses" the other component, it is understood that the one component may be directly connected to or may directly access the other component but unless explicitly described to the contrary, another component may be "connected" or "access" between the components.

In addition, terms including "unit," "er," "or," "module," and the like disclosed in the specification mean a unit that processes at least one function or operation and this may be implemented by hardware or software such as a processor, a micro processor, a micro controller, a central processing unit (CPU), a graphics processing unit (GPU), an accelerated processing unit (APU), a digital signal processor (DSP), an application specific integrated circuit (ASIC), and a field programmable gate array (FPGA) or a combination of hardware and software, and may be implemented in a form coupled to a memory that stores data necessary for processing at least one function or operation.

In addition, it is intended to clarify that the division of the components in the specification is only made for each main function that each component is responsible for. That is, two or more components to be described later below may be combined into one component, or one component may be divided into two or more components according to more subdivided functions. In addition, it goes without saying that each of the components to be described later below may additionally perform some or all of the functions of other components in addition to its own main function, and some of the main functions that each of the components is responsible for may be dedicated and performed by other components.

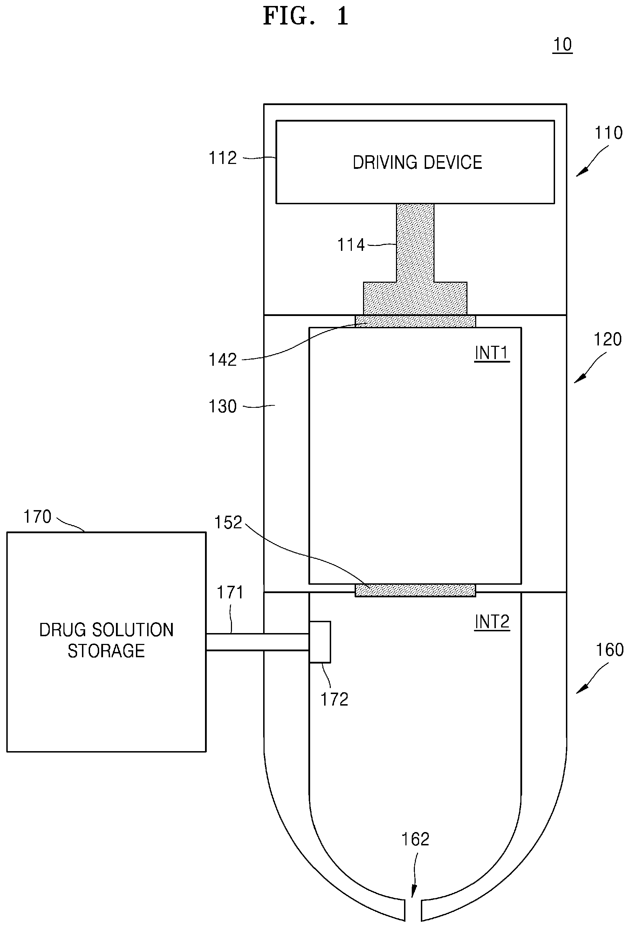

FIG. 1 is a conceptual diagram of a drug delivery device according to an embodiment of the present invention.

Referring to FIG. 1, the drug delivery device 10 may include a driving unit 110, a modular pressure transfer unit 120, a nozzle unit 160, and a drug solution storage 170.

The driving unit 110 may include a driving device 112 and a pressing portion 114 whose operation is controlled by the driving device 112.

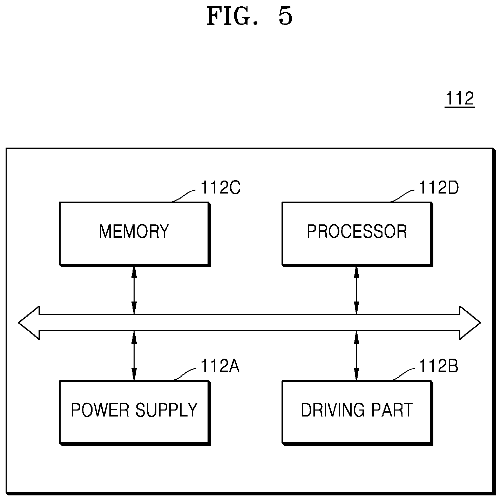

Detailed structure and operation of the driving device 112 will be described later with reference to FIG. 5.

The pressing portion 114 may move vertically up and down by the driving device 112.

According to an embodiment, the pressing portion 114 may be positioned such that a lower portion thereof is in close contact with the first elastic layer 142 of the modular pressure transfer unit 120 in an initial state.

According to an embodiment, the pressing portion 114 may apply pressure to the first elastic layer 142 of the modular pressure transfer unit 120 while moving by the driving device 112.

The modular pressure transfer unit 120 may be coupled to the driving unit 110 and may be detachable as needed.

The modular pressure transfer unit 120 may include a housing 130, a first elastic layer 142, and a second elastic layer 152.

The housing 130 forms a sealed internal space, and an inside INT1 thereof may be filled with a fluid. For example, the fluid may be a liquid such as water, or a fluid in a gel state.

The first elastic layer 142 may be formed of a material having elasticity. The first elastic layer 142 is coupled to the housing 130 and may transfer pressure applied from the driving unit 110 to the fluid inside INT1 of the housing 130.

The second elastic layer 152 may be formed of a material having elasticity. The second elastic layer 152 is coupled to the housing 130 and may transfer the pressure transferred through the fluid filled in the inside INT1 of the housing 130 to the drug solution inside INT2 of the nozzle unit 160.

According to an embodiment, the first elastic layer 142 and the second elastic layer 152 may be formed of the same material having elasticity, or may be formed of different materials.

According to an embodiment, each of the first elastic layer 142 and the second elastic layer 152 may be formed of a rubber material or a silicone material having elasticity.

The nozzle unit 160 may be coupled to the modular pressure transfer unit 120 and may be detachable as needed.

The nozzle unit 160 may inject the drug solution supplied from the drug solution storage 170 through a nozzle 162 using the pressure transferred by the modular pressure transfer unit 120.

According to an embodiment, the nozzle unit 160 may receive the drug solution from the drug solution storage 170 through a drug solution passage 171.

According to an embodiment, in order to prevent the drug solution from flowing backward from the nozzle unit 160 to the drug solution storage 170, a check valve 172 may be provided inside the drug solution passage 171 or at least one of both ends of the drug solution passage 171.

According to an embodiment, the drug solution supplied from the drug solution storage 170 is supplied to the inside INT2 of the nozzle unit 160, so that the inside INT2 of the nozzle unit 160 may be in a state filled with the drug solution.

According to an embodiment, a check valve (not shown) may be provided in a partial region of the nozzle 162 to prevent the drug solution ejected to the nozzle 162 from flowing backward into the nozzle unit 160.

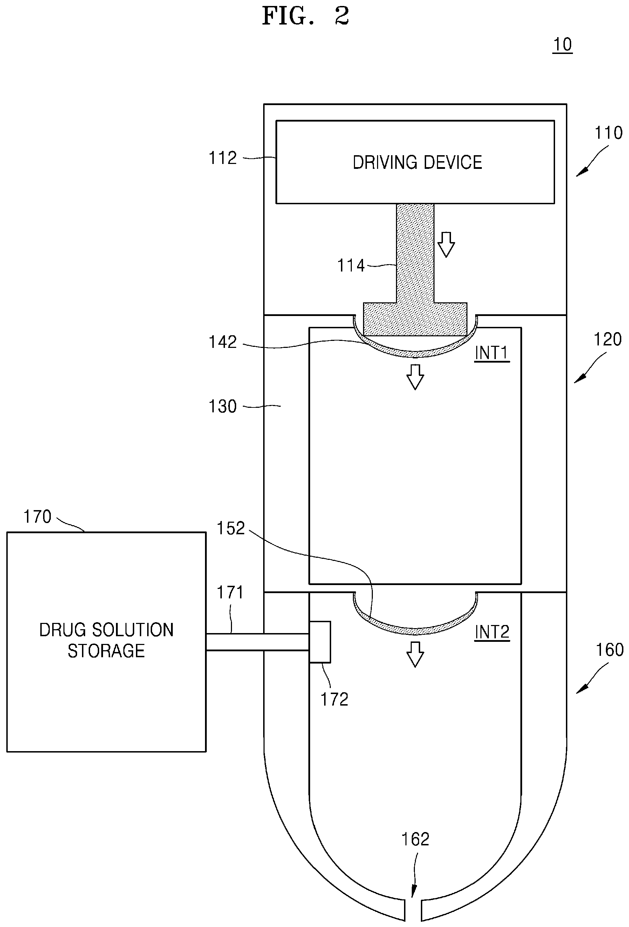

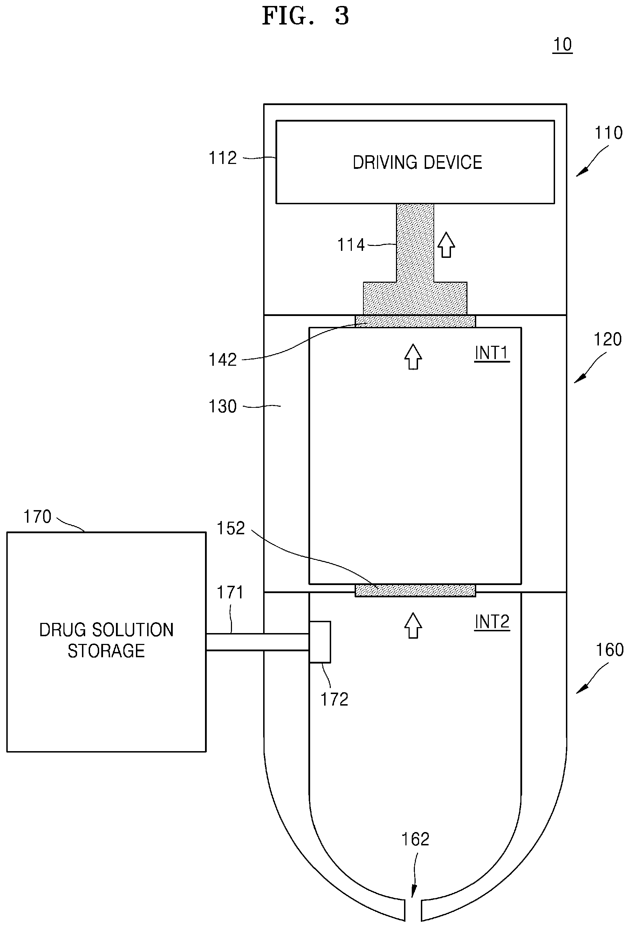

FIGS. 2 and 3 are diagrams for explaining an operation process of the drug delivery device shown in FIG. 1.

Referring to FIGS. 1 to 3, FIG. 2 schematically illustrates a state when ejecting a drug from the drug delivery device 10, and FIG. 3 schematically illustrates a state returned to the initial state after ejecting the drug from the drug delivery device 10.

Referring to FIG. 2, the pressing portion 114 applies downward pressure to the first elastic layer 142 while descending in the vertical direction by the driving device 112. Since the inside INT1 of the modular pressure transfer unit 120 is filled with the fluid, the pressure applied to the fluid is transferred to the second elastic layer 152 as the first elastic layer 142 expands toward an interior of the housing 130. The second elastic layer 152 may be expanded toward an exterior of the housing 130 by the pressure transferred through the fluid.

In FIG. 2, the lower surface of the pressing portion 114 and the first elastic layer 142 are shown as not being in close contact, but during actual operation, the pressing portion 114 may apply pressure to the first elastic layer 142 in a state where the lower surface is in close contact with the first elastic layer 142.

Since the inside INT2 of the nozzle unit 160 is filled with the drug solution, as the second elastic layer 152 expands toward an interior of the nozzle unit 160, the drug solution is ejected through the nozzle 162 by the pressure transferred by the second elastic layer 152.

Referring to FIG. 3, while the pressing portion 114 rises in the vertical direction by the driving device 112, the first elastic layer 142 returns to the state before the pressure was applied, and the second elastic layer 152 also returns to the state before the pressure was applied.

The drug delivery device 10 can inject the drug solution in the form of a jet through the nozzle 162 by rapidly repeating the states of FIG. 2 and FIG. 3.

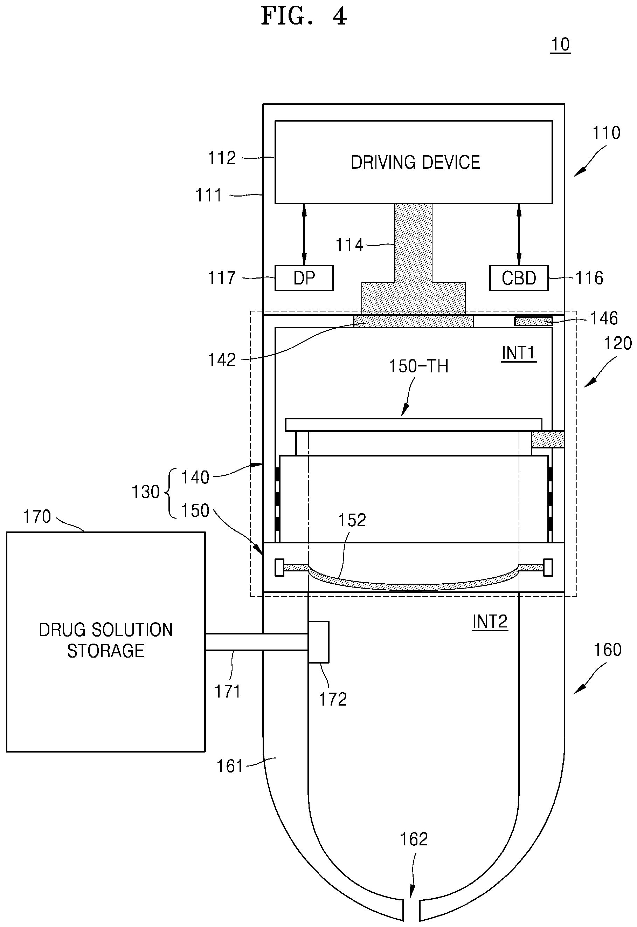

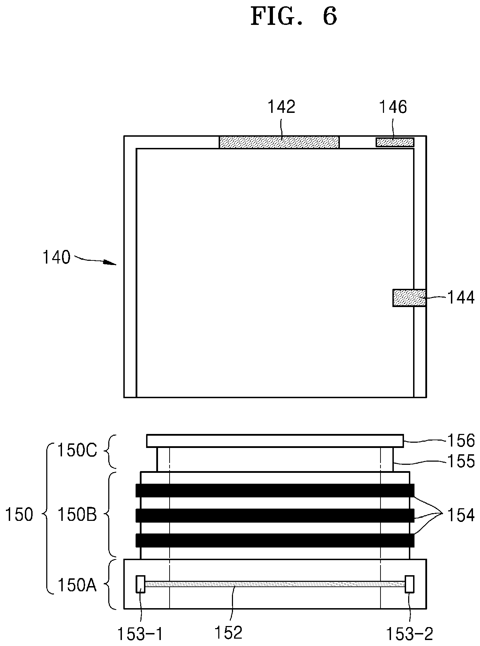

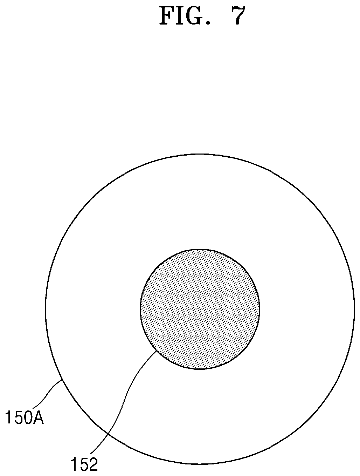

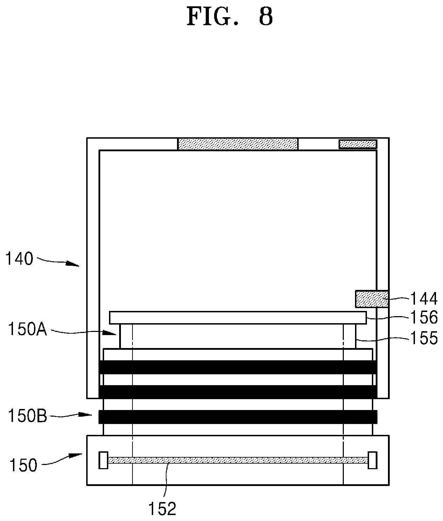

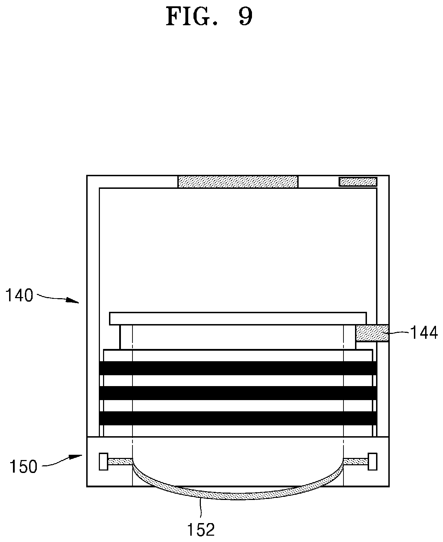

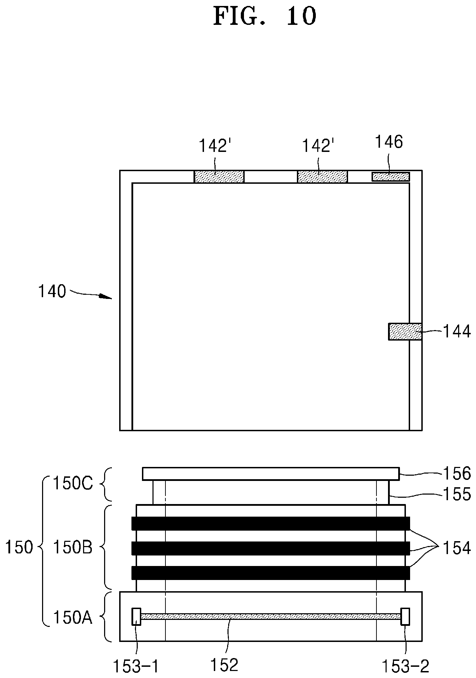

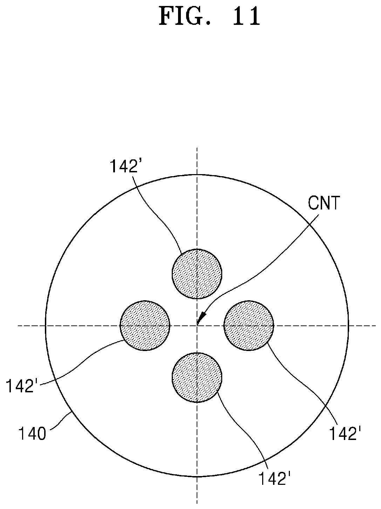

FIG. 4 is a diagram illustrating detailed components of the drug delivery device shown in FIG. 1. FIG. 5 is a block diagram according to an embodiment of the driving device shown in FIG. 4. FIGS. 6 and 7 are diagrams showing a detailed structure of the modular pressure transfer unit shown in FIG. 4. FIGS. 8 and 9 are diagrams showing states before use and a state for use of the modular pressure transfer unit shown in FIG. 6. FIGS. 10 and 11 are diagrams showing modified embodiments of the modular pressure transfer unit shown in FIG. 4.

Referring to FIG. 4, the drug delivery device 10 may include a driving unit 110, a modular pressure transfer unit 120, a nozzle unit 160, and a drug solution storage 170.

The driving unit 110 may include a driving device 112, a pressing portion 114 whose operation is controlled by the driving device 112, a coupling detection device 116, and a display 117.

Referring to FIG. 5, the driving device 112 may include a power supply 112A, a driving part 112B, a memory 112C, and a processor 112D.

The power supply 112A may supply power to respective components of the driving device 112.

According to an embodiment, the power supply 112A may supply power to components other than the driving device 112, for example, the coupling detection device 116 and the display 117.

The driving part 112B may move the pressing portion 114 under the control of the processor 112D.

According to an embodiment, the driving part 112B may move the pressing portion 114 up and down in the vertical direction.

According to an embodiment, the driving part 112B may be implemented in various forms for moving the pressing portion 114.

For example, the driving part 112B may include a motor and a configuration for converting rotational motion of the motor into vertical motion.

For example, the driving part 112B may include a solenoid coil structure, and in this case, the pressing portion 114 may include a magnetic material that receives a force of a magnetic field formed by the solenoid coil.

An upper portion of the pressing portion 114 may be connected to the driving part 112B of the driving device 112 or arranged in a position and structure capable of receiving the force applied by the driving part 112B.

A lower portion of the pressing portion 114 is in close contact with the first elastic layer 142, and the pressing portion 114 may apply pressure to the first elastic layer 142 toward an interior of the housing 130 of the modular pressure transfer unit 120.

The lower surface of the pressing portion 114 may be formed as a flat surface or a curved surface with a convex lower portion.

The memory 112C may store data necessary for the processing of the processor 112D for performing operations and functions of the driving device 112, and data generated during or after the processing of the processor 112D.

According to an embodiment, the memory 112C may store a program including program codes for performing the method of operating the drug delivery device according to an embodiment of the present invention, and the memory 112C may be coupled to the processor 112D to execute the program.

The processor 112D may perform data processing necessary for performing operations and functions of the driving device 112.

According to an embodiment, the processor 112D may control the driving part 112B.

Returning to FIG. 4, the coupling detection device 116 may detect a coupling state between the driving unit 110 and the modular pressure transfer unit 120.

According to an embodiment, the coupling detection device 116 may detect a coupling state with the driving unit 110 through a contact sensor (not shown) located at a portion connected to or in contact with the driving unit 110.

According to an embodiment, the coupling detection device 116 may collect identification information of the coupled modular pressure transfer unit 120. For example, the coupling detection device 116 may collect the identification information from the identification tag 146 of the modular pressure transfer unit 120. For example, the identification tag 146 may be implemented in a form capable of wirelessly transmitting stored identification information (e.g., an NFC tag).

The coupling detection device 116 may transmit information regarding a coupling state with the driving unit 110 (whether coupled or not) or the identification information of the coupled modular pressure transfer unit 120 to the driving device 112 side.

According to an embodiment, the information regarding the coupling state with the driving unit 110 or the identification information of the coupled modular pressure transfer unit 120 collected by the coupling detection device 116 may be displayed through the display 117.

The processor 112D of the driving device 112 may determine a usage amount of the corresponding modular pressure transfer unit 120 based on the coupling state with the unit 110 transmitted from the coupling detection device 116 or the identification information of the coupled modular pressure transfer unit 120.

According to an embodiment, when the modular pressure transfer unit 120 is detected as being coupled to the driving unit 110, the processor 112D may determine the usage amount of the modular pressure transfer unit 120 corresponding to the collected identification information. In this case, the usage amount may correspond to a cumulative value of pressure determined based on the number of times of pressing the first elastic layer 142 by the pressing portion 114 and a pressure at the time of pressing.

For example, the usage amount may correspond to a cumulative value of pressure applied each time the pressing portion 114 presses the first elastic layer 142, and the usage amount may be displayed through the display 117.

According to an embodiment, the processor 112D may determine whether the usage amount of the modular pressure transfer unit 120 currently in use exceeds a reference value, and may display the determination result through the display 117. In this case, the reference value may correspond to a replacement cycle according to durability of the modular pressure transfer unit 120.

The modular pressure transfer unit 120 may include a housing 130, a first elastic layer 142, and a second elastic layer 152.

The housing 130 forms a sealed internal space, and an inside INT1 thereof may be filled with a fluid.

According to an embodiment, the housing 130 may include a first sub-housing 140 in which the first elastic layer 142 is formed and a second sub-housing 150 in which the second elastic layer 152 is formed.

Referring to FIGS. 6 and 7, FIG. 6 shows a side view of a state where the first sub-housing 140 and the second sub-housing 150 are separated, and FIG. 7 shows a view of the second sub-housing 150 as viewed from below.

The first elastic layer 142 may be coupled to at least a portion (e.g., an upper surface) of the first sub-housing 140.

An identification tag 146 of the modular pressure transfer unit 120 may be included in a portion (e.g., the upper surface) of the first sub-housing 140.

A fixing pin 144 may be formed on an inner surface of the first sub-housing 140. The fixing pin 144 may fix the first sub-housing 140 in a state where an insertion portion 150B of the second sub-housing 150 is fully inserted into the inside of the first sub-housing 140.

The second sub-housing 150 may include a support portion 150A, an insertion portion 150B, and a fixing portion 150C.

The second elastic layer 152 may be formed inside the support portion 150A. The second elastic layer 152 may partition the fluid filled in the inside INT1 of the housing 130 of the modular pressure transfer unit 120 and the drug solution filled in the inside INT2 of the nozzle unit 160.

The support portion 150A may include fastening units 153-1 and 153-2 for fastening the second elastic layer 152 to the support portion 150A.

The insertion portion 150B is connected to the support portion 150A, and at least a part thereof may be inserted into the inside of the first sub-housing 140.

A packing portion 154 sealing a gap between the insertion portion 150B and an inner surface of the first sub-housing 140 may be formed on an outer surface of the insertion portion 150B.

According to an embodiment, the packing portion 154 may be composed of at least one O-ring.

The fixing portion 150C is connected to the insertion portion 150B, and may be caught by the fixing pin 144 in a state where the insertion portion 150B is fully inserted into the inside of the first sub-housing 140, and may be fixed in the fully inserted state by the fixing pin 144.

According to an embodiment, the fixing portion 150C may include an extension portion 155 connected to the insertion portion 150B, and a protrusion 156 connected to the extension portion 155 and forming a engagement protrusion on which the fixing pin 144 is caught.

According to an embodiment, a diameter of the extension portion 155 may be configured to be smaller than a diameter of the protrusion 156.

A through-hole 150-TH is formed along a vertical dotted line in FIG. 6 inside the support portion 150A, the insertion portion 150B, and the fixing portion 150C of the second sub-housing 150, and the through-hole 150-TH may be partitioned by the second elastic layer 152 included inside the support portion 150A.

Referring to FIGS. 8 and 9, FIG. 8 shows a state before use of the modular pressure transfer unit 120, and FIG. 9 shows a state for use of the modular pressure transfer unit 120.

Referring to FIG. 8, before use of the modular pressure transfer unit 120, the insertion portion 150B of the second sub-housing 150 is not fully inserted, and the protrusion 156 of the fixing portion 150A is not caught by the fixing pin 144. At this time, the inside of the modular pressure transfer unit 120 may be filled with a fluid (e.g., water).

Referring to FIG. 9, when using the modular pressure transfer unit 120, before coupling the modular pressure transfer unit 120 with the driving unit 110 and the nozzle unit 160, a user may push up the support portion 150 of the modular pressure transfer unit 120 to change the state such that the protrusion 156 of the fixing portion 150A is caught by the fixing pin 144.

Accordingly, the second elastic layer 152 may be convexly expanded downward by the fluid filled inside the modular pressure transfer unit 120. The second elastic layer 152 is expanded in a state where pressure is not applied from the outside of the modular pressure transfer unit 120, and when pressure is applied from the outside, it expands further downward to push out the drug solution filled in the inside INT2 of the nozzle unit 160. In this case, the second elastic layer 152 can more effectively transfer the pressure transferred from the outside to the drug solution side.

Referring to FIGS. 10 and 11, FIG. 10 is a side view of a modular pressure transfer unit to which another embodiment 142' of the first elastic layer is applied, and FIG. 11 is a view showing a first sub-housing 140 to which another embodiment 142' of the first elastic layer is applied, as viewed from above.

The first elastic layer 142' may be formed in a plurality of regions on an upper surface of the housing (e.g., the first sub-housing 140). According to an embodiment, the plurality of regions may be located at positions symmetric to each other with respect to a center point CNT of the upper surface of the housing (e.g., the first sub-housing 140).

According to an embodiment, the plurality of regions may be formed of an even number of regions symmetric to each other with respect to the center point CNT of the upper surface of the first sub-housing 140.

In this case, the pressing portion 114 of the driving unit 110 may be configured in the same number as the number of the plurality of regions.

Hereinabove, the present invention has been described with reference to the preferred embodiments. However, it will be appreciated by one of ordinary skill in the art that various modifications and changes of the present invention can be made without departing from the technical idea and the scope of the present invention which are defined in the appended claims and their equivalents.

## Claims

1. A modular pressure transfer unit comprising:
a housing configured to form a sealed internal space, wherein an inside of the housing is filled with a fluid;
a first elastic layer formed of a material having elasticity, coupled to the housing, and configured to transfer pressure applied from one side of an outside of the housing to the fluid; and
a second elastic layer formed of a material having elasticity, coupled to the housing, and configured to transfer the pressure transferred through the fluid to another side of the outside of the housing.

2. The modular pressure transfer unit of claim 1, wherein the housing comprises:
a first sub-housing in which the first elastic layer is formed; and
a second sub-housing in which the second elastic layer is formed.

3. The modular pressure transfer unit of claim 2, wherein the second sub-housing comprises:
a support portion in which the second elastic layer is formed;
an insertion portion connected to the support portion and configured such that at least a part thereof is inserted into an inside of the first sub-housing; and
a fixing portion connected to the insertion portion and configured to be caught and fixed by a fixing pin in a state where the insertion portion is fully inserted into the inside of the first sub-housing.

4. The modular pressure transfer unit of claim 3, wherein a packing portion configured to seal a gap between the insertion portion and an inner surface of the first sub-housing is formed on an outer surface of the insertion portion.

5. The modular pressure transfer unit of claim 3, wherein the fixing portion comprises:
an extension portion connected to the insertion portion; and
a protrusion connected to the extension portion and configured to form a engagement protrusion on which the fixing pin is caught.

6. The modular pressure transfer unit of claim 1, wherein the first elastic layer is configured to expand toward an interior of the housing by the pressure applied from the outside of the housing, and wherein the second elastic layer is configured to expand toward an exterior of the housing by the pressure transferred through the fluid.

7. The modular pressure transfer unit of claim 1, further comprising:
an identification tag configured to store identification information of the modular pressure transfer unit.

8. The modular pressure transfer unit of claim 1, wherein the first elastic layer is formed in a plurality of regions on an upper surface of the housing.

9. The modular pressure transfer unit of claim 8, wherein the plurality of regions are located at positions symmetric to each other with respect to a center point of the upper surface of the housing.

10. A drug delivery device comprising:
a driving unit;
a modular pressure transfer unit coupled to the driving unit and configured to transfer pressure applied from the driving unit; and
a nozzle unit coupled to the modular pressure transfer unit and configured to inject a drug solution using the pressure transferred by the modular pressure transfer unit,
wherein the modular pressure transfer unit comprises:
a housing configured to form a sealed internal space, wherein an inside of the housing is filled with a fluid;
a first elastic layer formed of a material having elasticity, coupled to the housing, and configured to transfer the pressure applied from the driving unit to the fluid; and
a second elastic layer formed of a material having elasticity, coupled to the housing, and configured to transfer the pressure transferred through the fluid to the drug solution inside the nozzle unit.

11. The drug delivery device of claim 10, wherein the driving unit comprises:
a pressing portion configured to apply pressure to the first elastic layer; and
a driving device configured to control an operation of the pressing portion.

12. The drug delivery device of claim 11, further comprising: a coupling detection device configured to detect a coupling state between the driving unit and the modular pressure transfer unit.

13. The drug delivery device of claim 12, wherein the coupling detection device is configured to collect identification information of the coupled modular pressure transfer unit.

14. The drug delivery device of claim 13, wherein the driving device is configured to, when the modular pressure transfer unit is detected as being in a coupled state by the coupling detection device, receive the collected identification information and determine a total usage amount of the modular pressure transfer unit corresponding to the identification information.

15. The drug delivery device of claim 14, wherein the total usage amount of the modular pressure transfer unit corresponds to a cumulative value of pressure determined based on a number of times of pressing the first elastic layer by the pressing portion and a pressure at a time of pressing.
